# EUROPEAN PATENT APPLICATION

(11) **EP 2 283 890 A1**
(43) Date of publication of application: **16.02.2011**
(21) Application number: 08722577.7
(22) Date of filing: 21.03.2008
(51) Int. Cl.: A61M 25/00

(54) **CATHETER BALLOON ASSEMBLY**

(71) Applicant: NIPRO CORPORATION, Kita-ku Osaka-shi, Osaka 531-8510 (JP)
(72) Inventor: MASUDA, Toshiaki, Osaka-shi Osaka 531-8510 (JP); MOMOTANI, Shinji, Osaka-shi Osaka 531-8510 (JP)
(74) Representative: Merrifield, Sarah Elizabeth
(86) International application number: PCT/JP2008/055213
(87) International publication number: WO 2009/116159

(57) **Abstract**

A dilation-assisting member is easily disposed at a gap between a balloon and a site to be dilated and does not drop off even if the balloon is expanded extremely exceeding a predetermined diameter.

In a balloon assembly for a catheter 1 having, on an external surface of a balloon 3_{;} the dilation-assisting member 4 which dilates a stenosis site the dilation-assisting member is extended from a proximal end portion to a distal end portion of the balloon and one end of the dilation-assisting member is fixed to a first support member 5 provided at the proximal end portion and the other end of the dilation-assisting member is held slidably by a second support member 6 provided at the distal end portion, and the other end of the dilation-assisting member is protruded from the second support member such that the dilation-assisting member 4 has a length which enables the dilation-assisting member to extend from the proximal end portion to the distal end portion even when the balloon 3 is expanded.

## Description

### Technical Field

The present invention is related to a balloon assembly for a catheter, and particularly the balloon assembly for the catheter which enables a balloon portion to be expanded easily in a stiff lesion.

### Background Art

An inflatable balloon catheter with a balloon that is inserted in a blood vessel and expanded to dilate the blood vessel has been known as a medical device for treating occlusion or stenosis of the blood vessel of a patient has been known. A desired size and physical properties of this balloon depend on the size of the blood vessel of the patient and the use of the balloon.

Further, when the dilation is difficult by using the prior art inflatable balloon catheter, a technique of inserting a wire between the balloon and the site to be dilated or a technique using a cutting edge attached on an external surface of the balloon has been employed in order that the dilation rate (or degree) is increased. Furthermore, a balloon catheter provided with the cutting edge on the external surface of the balloon was suggested, wherein the cutting edge is oscillated to improve the dilation effect (see, for example, Patent Document 1).

Although the balloon catheter of a construction wherein the cutting edge is merely attached to the external surface of the balloon is effective to a predetermined diameter, there is a possibility of dropping-off of the cutting edge when the balloon is expanded extremely beyond the predetermined diameter. Further, the treatment in a curved site using the catheter of the construction wherein the cutting edge is merely attached to the external surface of the balloon, is difficult.
[Patent Document 1] JP 07-67967 A

### Disclosure of Invention

### Problems to be Solved by Invention

In order to solve the above problem, the object of the present invention is to provide a balloon assembly for a catheter, which is provided with a dilation-assisting member that is easily positioned in a gap between a balloon and a site to be dilated even in a curved site and does not drop off even when the balloon is expanded extremely over a predetermined diameter.

### Means to Solve Problems

In order to achieve the object, the present invention is a catheter balloon including, on an external surface of of a balloon, a dilation-assisting member for dilating a stenosis site, which is characterized in that:
the dilation-assisting member extends from a proximal end portion to a distal end portion of the balloon;
the dilation-assisting member is held slidably by a first support member provided at the proximal end portion and a second support member provided at the distal end portion; and
an end portion of the dilation-assisting member is protruded from at least one of the first support member and the second support member such that the dilation-assisting member has a length extending from the proximal end portion to the distal end portion of the balloon even when the balloon is expanded.

In other words, the present invention provides a balloon assembly for a catheter having a balloon and a dilation-assisting member(s) for dilating a stenosis site which member is provided on the external surface of the balloon, which is characterized by a construction in which the assembly is provided with one or more dilation-assisting members extending from a proximal end portion of the balloon to a distal end portion of the balloon, a first support member and a second support member for holding the dilation-assisting member(s), which are arranged in the proximal end portion and the distal end portion of the balloon respectively, and
at least one end portion of the dilation-assisting member is held slidably by at least one of the first support member and the second support member and the at least one end portion is protruded from the at least one of the first and the second support members such that the dilation-assisting member has a length extending from the proximal end portion to the distal end portion of the balloon even when the balloon is expanded.

The balloon may be formed from a known material. The representative materials include a polyethylene terephthalate, naylon, a polyolefin such as a polyethylene, a polypropylene and so on, a thermoplastic elastomer such as a polyamide elastomer, a silicone rubber and a latex rubber. The use of polyethylene terephthalate or nylon is desirable.

The dilation-assisting member is attached to the external surface of the balloon and the number of the members is not limited to a particular one. One or more members or many members, for example, six to eight members may be used. Typically, the construction is preferable wherein four dilation-assisting members are provided at predetermined intervals on the external surface of the balloon, whereby the stenosis site in the blood vessel can be dilated (or expanded).

The dilation-assisting member may be formed from a known material which is elastically deformable. A nickel-titanium alloy can be exemplified as a representative material. Further, the dilation-assisting member may be of a double structure having an inner core and an outer sheath, wherein the core is formed from platinum and the sheath is formed from the nickel-titanium alloy, for the purpose of increasing the contrast of the member.

The first support member and the second support member are formed from a rigid material such as a resin, a metal, or an alloy. A stainless steel such as SUS304 is preferably used as the material therefor. The first and the second support members are provided at the proximal end portion and the distal end portion of the balloon respectively and fixed thereto with an adhesive. An epoxy-based adhesive, an acryl-based adhesive, a cyanoacrylate-based adhesive or a polyurethane-based adhesive is preferably used as the adhesive.

In the balloon assembly for catheter of the above-described construction according to the present invention, at least one end of the dilation-assisting member provided on the external surface of the balloon is held slidably by the first or the second support member. For this reason, even if the balloon is repeatedly expanded and deflated, the dilation-assisting member always expands and contracts correspondingly to the displacement of the balloon and extends from the proximal end portion to the distal end portion of the balloon, whereby the dilation-assisting member does not drop off.

In one embodiment of the present invention, one end of the dilation-assisting member is fixed to the first support member and the other end is held slidably by the second support member and penetrates the second support member to protrudes from the second support member. Further, said other end of the dilation-assisting member may be provided with an engagement portion which is engaged to a through hole of the support member such that the dilation-assisting member is prevented from slipping off from the through hole of the second support member. The engagement portion may be formed by deforming or curving said other end of the dilation-assisting member, or may be formed by providing a protrusion or a head at said other end.

According to the present invention having the above-described construction, even if the inflation and the deflation are repeated in the blood vessel, the dilation-assisting member mounted on the external surface of the balloon does not drop off from the balloon since the dilation-assisting member is held at its one end by the first support member, and held slidably at its other end by the second support member.

Another embodiment of the present invention is characterized in that the dilation-assisting member is made of an elastically deformable wire with a narrow edge portion that is polished sharply, and a through hole through is provided in the second support member, through which the wire freely passes while the narrow edge portion faces outwardly.

According to the present invention having the above-described construction, the use of the elastically deformable wire member makes it possible to give the dilation-assisting member which easily follows the inflation and the deflation of the balloon. Further, the narrow edge portion obtained by polished sharply enables the stiff leision to be dilated surely. Furthermore, since the wire can be guided by the through hole in the second support member, the wire can surely slide correspondingly to the inflation and deflation of the balloon.

Another embodiment of the present invention is characterized in the dilation-assisting member is of a nickel-titanium alloy.

According to the present invention of the above-described construction, the stenosis blood vessel can be surely dilated since the dilation-assisting member is made of a metal. Further, the dilation-assisting member which can surely contract when the balloon is deflated can be formed since the dilate-assisting member is formed from a shape memory metal which keeps a predetermined shape.

Another embodiment of the present invention is characterized by a construction wherein the second support member is a ring shape and four through holes are provided peripherally to the second support member at equal intervals and four dilation-assisting members are provided on the external surface of the balloon at equal intervals.

According to the present invention of the above-described construction, the internal portion of the stenosis blood vessel can be dilated uniformly. Further, since the respective dilation-assisting members are independently slidable, the balloon catheter can be used in the curved site.

Another embodiment of the present invention is characterized in that a protection cover is provided at a position distal to the second support member for covering the second support member and the other end of the dilation-assisting member together. The material for the protection cover is preferably a polyolefin such as a polyethylene and a polypropylene and so on, a polyvinyl chloride, a polyurethane, a thermoplastic elastomer such as a polyamide elastomer, a silicone rubber or a latex rubber, similarly to the material for the balloon.

According to the present invention of the above-described construction, the balloon catheter provided with the dilation-assisting member that is safely passed through the blood vessel can be made, and the inner wall of the blood vessel is not damaged when the catheter is withdrawn after the treatment.

According to another embodiment of the present invention, a balloon catheter including the above-described balloon assembly for the catheter and a catheter tube is provided. This balloon catheter is constructed by inserting the catheter tube in the balloon to a predetermined position and bonding or welding the both ends of the balloon to the catheter tube. In this case, an epoxy-based adhesive, an acrylic adhesive or a cyanoacrylate-based adhesive is desirably used as the adhesive for bonding both members.

### Effect of the Invention

The present invention is constructed such that the dilation-assisting member is extended from the proximal end portion to the distal end portion of the balloon and the dilation-assisting member is slidably held by the first support member provided at the proximal end portion and the second support member provided at the distal end portion and the dilation-assisting member is disposed from the proximal end portion to the distal end portion of the balloon even if the balloon is expanded. This construction can give the balloon assembly for catheter which is provided with the dilation-assisting member that is positioned at the gap between the balloon and the site to be dilated even in the curved site and follows the inflation and the deflation of the balloon and does not drop off even if the balloon is extremely expanded exceeding a predetermined diameter.

### Brief Description of Drawings

Fig. 1 is a schematic explanatory diagram showing an example of a catheter balloon according to the present invention, and Fig. 1a shows a state wherein the balloon is deflated and Fig. 1b shows a state wherein the balloon is expanded.

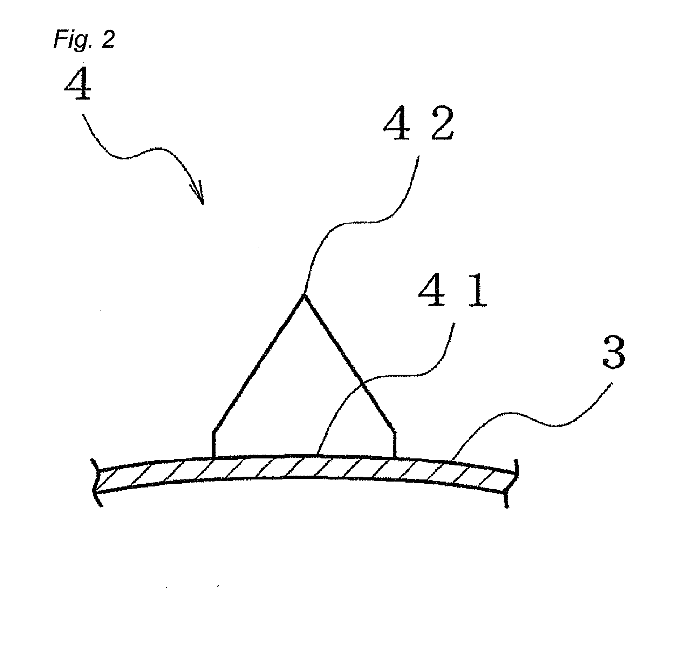

Fig. 2 is an enlarged cross-sectional view of a dilation-assisting member shown in Fig. 1.
Fig. 3 is a cross-sectional view of a second support member for the dilation-assisting member shown in Fig. 1.

### Description of Reference Numeral

- 1: Balloon assembly for a catheter
- 2: Catheter tube
- 3: Balloon
- 4: Dilation-assisting member
- 4a: Tip
- 5: First support member
- 6: Second support member
- 6a: Through hole
- 7: Protection cover
- 42: Narrow edge portion

### Best Modes for Carrying Out the Invention

Embodiment of the balloon assembly for a catheter according to the present invention is described in detail referring to the drawings below.

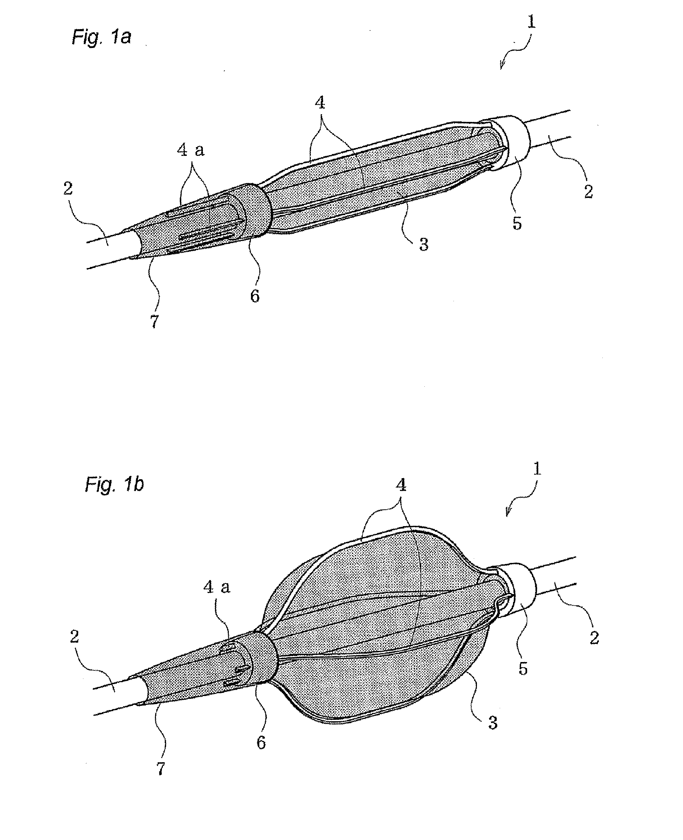

As shown in Fig. 1a, a balloon assembly for a catheter 1 according to the present invention is of a construction which has a catheter tube 2 and a balloon 3, wherein a dilation-assisting member 4 is attached by means of a first support member 5 provided at a proximal end portion of the balloon 3 and a second support member 6 provided at a distal end portion. Both of these first and second support members 5 and 6 are a ring of a rigid body (for example, a metal). Therefore, the balloon catheter is given of which construction includes the balloon assembly for catheter 1 and the catheter tube 2.

The dilation-assisting member 4 is held slidably by the first support member 5 and the second support member 6. This construction is preferable since it realizes a construction wherein the dilation-assisting member 4 extends from the proximal end portion to the distal end portion of the balloon without dropping off from the balloon 3 even when the balloon 3 is expanded.

In this embodiment, one end of the dilation-assisting member 4 is fixed to the first support member 5 and the other end thereof is held slidably by the second support member 6. Further, a tip 4a of the other end of the dilation-assisting member 4 has a length such that it protrudes outwardly from the second support member 6.

Further, as shown in Fig. 1b, the dilation-assisting member 4 has a length such that the tip 4a of the dilation-assisting member 4 protrudes outwardly to some extent from the second support member 6 even if the balloon 3 is expanded maximally, whereby a construction wherein the dilation-assisting member 4 always extends from the proximal end portion to the distal end portion of the balloon 3 is realized.

In addition, a protection cover 7 is provided at the position distal to the second support member 6 for covering the second support member 6 and the tip 4a of the protruded portion of the dilation-assisting member 4 together.

This balloon catheter becomes one which is provided with the dilation-assisting member that can safely pass through the blood vessel and the catheter does not damage the inner wall of the blood vessel when being withdrawn after the treatment, since the entire is covered by the resin protection cover 7 in even the construction wherein the tip 4a is protruded from the second support member 6.

As described above, this balloon 3 can dilate the stenosis site of the blood vessel when it is inserted into the blood vessel and expanded, since the dilation-assisting member 4 is mounted on the external surface of the balloon 3. Further, since the construction become one wherein the both ends of the dilation-assisting member 4 are supported, the dilation-assisting member 4 does not drop off even if the inflation and the deflation are repeated in the blood vessel.

The dilation-assisting member 4 is a wire which is elastically deformable. Further, the shape of the wire is made to be one which has a shallow curved portion 41 or a linear portion adjacent to the balloon 3 and a narrow edge portion 42 that is polished and outwardly sharpened. Therefore, the dilation-assisting member 4 can be obtained which easily follows the inflation and the deflation of the balloon 3 and dilates the stenosis site such that the stenosis site is incised when the balloon 3 is expanded.

Further, a plurality of dilation-assisting members 4 are provided on the external surface of the balloon. In this embodiment, four dilation-assisting members 4 are provided peripherally to the second support member at equal intervals. The construction wherein four dilation-assisting members 4 are provided on the outer circumference of the balloon makes it possible to dilate the stenosis site in the blood vessel.

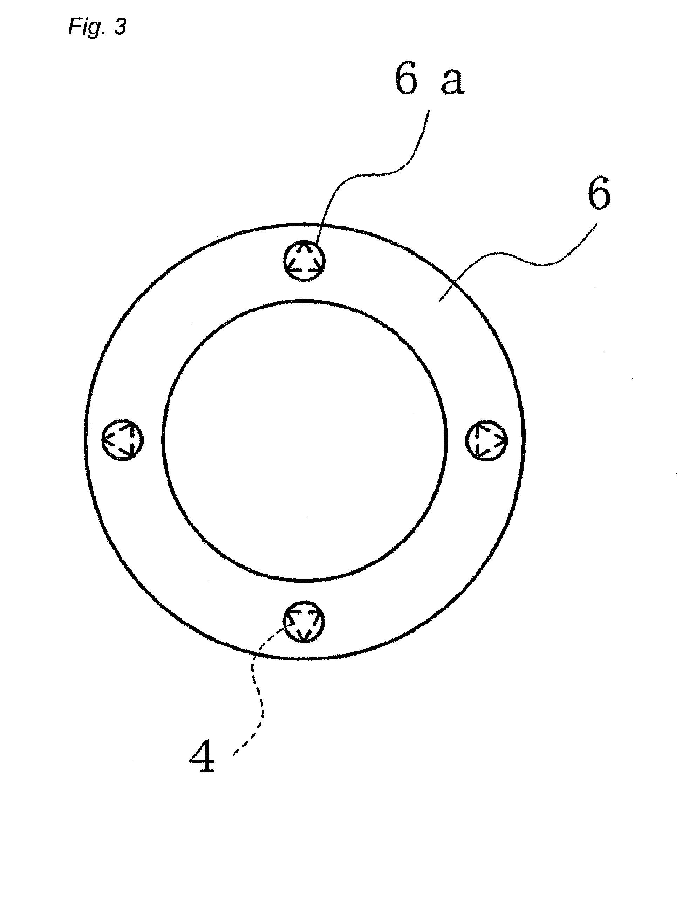

An example is shown in Fig. 3, wherein four dilation-assisting members 4 are mounted. This is an example wherein four through holes 6a are provided in the periphery portion of the second support member 6 at equal intervals and four dilation-assisting members 4 are supported slidably. Further, since these four dilation-assisting members 4 can freely slide independently from each other, this catheter balloon becomes one which can be used in the curved site. Furthermore, the blood vessel can be dilated uniformly since the stenosis blood vessel is dilated being incised in four direction in the inside of the blood vessel simultaneously with the inflation operation of the balloon 3 in the stenosis blood vessel.

The second support member 6 is preferably formed from a metal. This is because the member 6 is prevented from being damaged when the dilation-assisting member 4 slides in the through hole 6a. Further, the dilation-assisting member 4 is of a nickel-titanium alloy.

In the case where the dilation-assisting member 4 is formed from a nickel-titanium alloy, the member can memorize a shape when the balloon contracts since the nickel-titanium alloy is a shape memory metal which maintains a predetermined shape. This construction makes it possible to form the dilation-assisting member which can follow an inflation force to dilate by deforming elastically upon the inflation of the balloon 3 and easily return to the memorized shape using an elastic force for recovering to surely contract correspondingly to the contraction of the balloon 3. Further, the balloon assembly for catheter can surely dilate the stenosis site since the dilation-assisting member is of the metal wire.

The above-described construction makes it possible to easily expand and deflate the balloon 3 after the balloon assembly for catheter 1 equipped with a predetermined number of dilation-assisting members on the periphery is delivered to the stenosis site in the blood vessel. Further, the balloon 3 can easily expand and deflate at a site where the blood vessel is curved, fitting the curved shape. Furthermore, the dilation-assisting member 4 does not drop off even if the balloon 3 expands extremely over the predetermined diameter.

As described above, since the present invention is constructed so that the dilation-assisting member is extended from the proximal end portion to the distal end portion of the balloon outer circumference and the dilation-assisting member is held slidably by the first support member provided at the proximal end portion and the second support member provided at the distal end portion and the dilation-assisting member extends from the proximal end portion to the distal end portion of the balloon when the balloon is expanded, the balloon assembly for catheter can be obtained which is equipped with the dilation-assisting member that is disposed at the gap between the balloon and the site to be expanded even in the curved site and easily follows the inflation and deflation of the balloon without dropping off even if the balloon is expanded extremely over the predetermined diameter.

Further, the wire-like dilation assisting member of the nickel-titanium alloy which is a shape memory metal makes it possible to give the balloon assembly for catheter, which elastically deforms so as to surely dilate the stenosis site in the blood vessel and easily follow the inflation and deflation of the balloon.

## Claims

1. A balloon assembly for a catheter comprising, on an external surface of the balloon, a dilation-assisting member for dilating a stenosis site, which is **characterized in that**:
the dilation-assisting member extends from a proximal end portion to a distal end portion of the balloon;
the dilation-assisting member is held slidably by a first support member provided at the proximal end portion and a second support member provided at the distal end portion; and
an end portion of the dilation-assisting member is protruded from at least one of the first support member and the second support member such that the dilation-assisting member has a length extending from the proximal end portion to the distal end portion of the balloon even when the balloon is expanded.

2. The balloon assembly for the catheter according to claim 1, which is **characterized in that** one end of the dilation-assisting member is fixed to the first support member and the other end of the dilation-assisting member is held slidably by the second support member provided at the distal end portion, and said other end of the dilation-assisting member is protruded from the second support member.

3. The balloon assembly for the catheter according to claim 1 or 2, which is **characterized in that** the dilation-assisting member is made of an elastically deformable wire having a narrow edge portion which is polished sharply, and a through hole is provided in the second support member, through which the wire freely passes while the narrow edge portion faces outwardly.

4. The balloon assembly for the catheter according to any one of claims 1 to 3, which is **characterized in that** the dilation-assisting member is made of a nickel-titanium alloy.

5. The balloon assembly for the catheter according to any one of claims 1 to 4, which is **characterized in that** the assembly is constructed such that the second support member is of a ring shape, and four said through holes are provided peripherally to the second support member at equal intervals and four said dilation-assisting members are provided on the external surface of the balloon.

6. The balloon assembly for the catheter according to any one of claims 2 to 5, which is **characterized in that** a protection cover is attached at a position distal to the second support member for covering the second support member and said other end of the dilation-assisting member together.

7. A balloon catheter which is **characterized by** comprising the balloon assembly for the catheter according to any one of claims 1 to 6 and a catheter tube.
